# EUROPEAN PATENT SPECIFICATION

(11) **EP 1 745 021 B1**
(45) Date of publication and mention of the grant of the patent: **11.11.2009**
(21) Application number: 05740294.3
(22) Date of filing: 26.04.2005
(51) Int. Cl.: C07D 209/48, C07D 403/04

(54) **HYDROFORMYLATION PROCESS FOR PHARMACEUTICAL INTERMEDIATE**
HYDROFORMYLIERUNGSVERFAHREN FÜR EIN PHARMAZEUTISCHES ZWISCEHNPRODUKT
PROCÉDÉ D"HYDROFORMYLATION D"INTERMÉDIAIRES PHARMACEUTIQUES

(30) Priority: 29.04.2004 US 566471 P
(43) Date of publication of application: 24.01.2007
(73) Proprietor: Dr. Reddy's Laboratories (EU) Limited, Beverley East Yorkshire HU17 0LD (GB)
(72) Inventor: DAUGS, Edward, D., Midland, MI 48642 (US); PENG, Wei-Jun, Midland, Michigan 48642 (US); RAND, Cynthia, L., Sanford, MI 48657 (US)
(74) Representative: Dey, Michael
(86) International application number: PCT/US2005/014349
(87) International publication number: WO 2005/110986

(56) References cited:
- FERNANDEZ, M. M. ET AL: "Enzymic synthesis of peptides containing unnatural amino acids" ENZYME AND MICROBIAL TECHNOLOGY , 17(11), 964-71 CODEN: EMTED2; ISSN: 0141-0229, 1995, XP008048929
- KAZMAIER, ULI: "Introduction of Allylic Side Chains onto Peptides by Pd(0)-Catalyzed "Ester Enolate Claisen Rearrangement"" JOURNAL OF ORGANIC CHEMISTRY , 59(22), 6667-70 CODEN: JOCEAH; ISSN: 0022-3263, 1994, XP008048930
- HORGAN, STEPHEN W. ET AL: "Process Development in the Synthesis of the ACE Intermediate MDL 28,726" ORGANIC PROCESS RESEARCH & DEVELOPMENT, 3(4), 241 247 CODEN: OPRDFK; ISSN: 1083-6160, 1999, XP008048935 cited in the application
- FLYNN, GARY A. ET AL: "An acyl-iminium ion cyclization route to a novel conformationally restricted dipeptide mimic: applications to angiotensin-converting enzyme inhibition" JOURNAL OF THE AMERICAN CHEMICAL SOCIETY, 109(25), 7914 -15 CODEN: JACSAT; ISSN: 0002-7863, 1987, XP008048936 cited in the application
- OJIMA, IWAO ET AL: "New and Efficient Route to Pipecolic Acid Derivatives by Means of Rh-Catalyzed Intramolecular Cyclohydrocarbonylation" JOURNAL OF ORGANIC CHEMISTRY, 60(22), 7078 -9 CODEN: JOCEAH; ISSN: 0022-3263, 1995, XP008048939 cited in the application
- TEOH, EUNEACE ET AL: "One-pot tandem enantioselective hydrogenation-hydroformylation synthesis of cyclic .alpha.-amino acids" NEW JOURNAL OF CHEMISTRY, 27(2), 387 -394 CODEN: NJCHE5; ISSN: 1144-0546, 2003, XP008048938 cited in the application

## Description

### Field of the invention

The invention relates to an improved process for the preparation of an advanced synthetic intermediate of ACE inhibitors.

### Background to the invention

The tricyclic acid MDL 28,726 (**1**) is a key intermediate in the synthesis of ACE inhibitors MDL 27,210, MDL 100,240 and related analogues, which also possess inhibition activity against neutral endopeptidase (NEP). There is a requirement for an improved synthetic route to MDL 28,726 that provides favourable process economics for large scale commercial operation; this problem is addressed by the present invention.

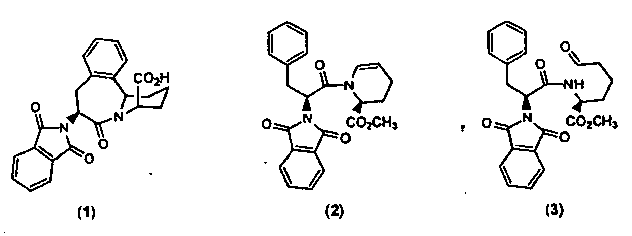

The original synthetic route to (**1**), reported by Flynn et al. (J. Am. Chem. Soc., 1987, 109, 7914) culminates in a stereoselective acyl-iminium ion induced cyclization to form the tricyclic ring system of (**1**). This reaction also forms the basis of an improved route reported by Horgan et al. (Org. Proc. Res. Dev., 1999, 3, 241), in which the desired stereoisomer of the cyclization substrate (**2**) is prepared more efficiently via an enzymatic resolution of a hydroxynorleucine derivative early in the synthesis. In contrast, the Flynn synthesis requires preparative HPLC separation of a 1:1 mixture of (**2**) and its opposite diastereoisomer and also requires a low temperature ozonolysis step. Although demonstrated at on pilot plant scale, the Horgan synthesis has certain features which render it unsuitable for commercial operation. In particular the route requires a low temperature Swern oxidation to produce (**2**) via the intermediate aldehyde (**3**), which is not ideal for large scale preparations, as it typically involves cryogenic reaction conditions, control of dimethylsulfide by-product emissions, expensive reagents such as oxalyl chloride and variable yields. 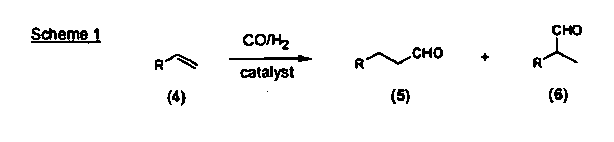 Hydroformylation of monosubstituted olefins (**4**; also known as α-olefins), catalyzed by group VIII transition metal complexes of phosphorus containing ligands, is a synthetically useful reaction, provided that high selectivity between the linear (**5**) and branched (**6**) aldehyde products can be achieved (Scheme 1). Typically, it is preferable that the ratio of the desired product to its regioisomer is at least 80:20, more preferably at least 90:10. In an ideal case, complete regioselectivity is achieved in combination with efficient substrate conversion. For cases where the linear regioisomer (**5**) is desired, a number of different catalysts have been designed for this purpose (for a review, see Recent Advances on Chemo-, Regio- and Stereoselective Hydroformylation, Breit and Seiche, Synthesis, 2001, 1, pp 1-36). Rhodium complexes of bisphosphite ligands provide one of the best known classes of linear-selective hydroformylation catalysts (US4668651 and US4769498). Representative ligands from this class include BIPHEPHOS (7) and the bisphosphite (**8**). A more recent series of bis-chelating ligands designed for linear selective hydroformylation is reported in WO2004035595; rhodium complexes of these ligands give particularly high linear:branched product ratios for simple α-olefins such as 1-octene and high catalytic activity to enable efficient substrate conversion at low catalyst loading.

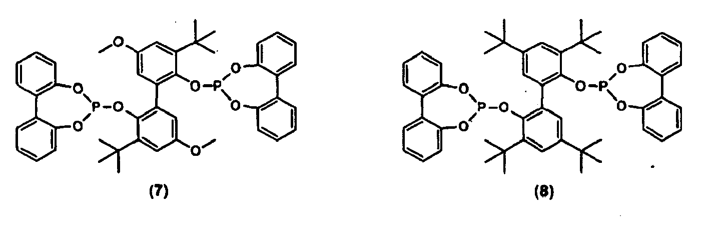

The methodology for hydroformylation of monosubstituted olefins was originally designed for relatively simple, unfunctionalized olefins such as 1-alkenes, e.g. 1-propene, 1-octene and styrene. Subsequently, a number of applications to more complex α-olefins, leading to higher value products, have been reported. As the structural complexity of an α-olefin increases, for example through the presence of functional groups (Cuny and Buchwald, J. Am. Chem. Soc., 1993, 115, 2066), subtle changes in the substrate can have a profound effect on the selectivity that is achievable with a given hydroformylation catalyst. Thus, the identity of a suitable catalyst for a particular substrate becomes much less predictable. For example, this is evident by comparing a process reported by Ojima et al (J. Org. Chem., 1995, 60, 7078) with another reported by Teoh et al (New. J. Chem., 2003, 27, 387). In the Ojima example [Reaction (a) in Scheme 2], a Rh-(BIPHENPHOS) catalyst provides 100% regioselectivity for the allyl glycinate substrate (**9**). In this process, the initially formed linear aldehyde, corresponding to (**5**) in Scheme 1, undergoes spontaneous cyclization to form a heterocyclic product. In the Teoh example [Reaction (b) in Scheme 2] use of the same catalyst on substrate (**10**), differing from (**9**) only in the nature of ester and N-acyl groups, a 2:1 mixture of regioisomers is produced. Because of the formation of a large amount of branched regioisomer requiring separation from the desired linear regioisomer, Reaction (b) is not a synthetically useful process.

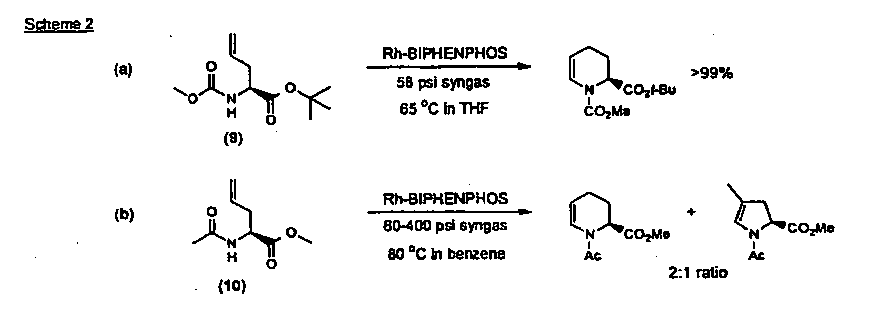

### Summary of the invention

In one aspect, the present invention is based on a novel process for the preparation of an aldehyde of formula (**I**), wherein (N)_{PrG} is a protected amino group, R is an alkyl or aralkyl group and each of X¹⁻⁴ is independently H or a non-reacting substituent, which comprises hydroformylation of an α-olefin of formula (**II**), by reaction with syngas (CO/H₂) in the presence of, as catalyst, a group VIII transition metal complex of a phosphorus-containing ligand. Aldehyde (**I**), the product of linear hydroformylation, is formed in preference to aldehyde (**III**). Optional recovery and efficient recycle of the intact hydroformylation catalyst and the ease of direct product isolation further characterize the operation of this manufacturing process.

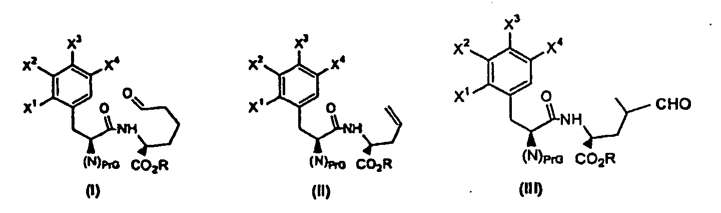

In another aspect of the present invention, the process to convert (**II**) to (**I**) further enables a novel and efficient manufacturing route to MDL 28,726 and analogues, as key precursors to dual ACE-NEP inhibitors. Prior to this invention, the preferred methods of preparation for such bioactive compounds would have used a protracted linear synthesis via acetal-protected L-allysine (for representative references, see US6174707, US5508272 and US6166227), or a hydroxynorleucine derviative with a subsequent oxidation to the aldehyde as described by by Horgan et al. (Org. Proc. Res. Dev., 1999, 3, 241).

In yet another aspect of the present invention, the α-olefin (**II**) is a novel composition.

### Description of the invention

In the hydroformylation process of the invention, R in compounds (**I**) and (**II**) is selected preferably from the group consisting of methyl; ethyl, *n*-propyl, *n*-butyl, benzyl and benzhydryl. More preferably, R is methyl. Preferably, the protected amino group (N)_{PrG} is chosen to be stable to acid treatment. More preferable (N)_{PrG} is a cyclic imide and most preferably it is *N*-phthalimide. Commonly, each of X¹⁻⁴ is H although it will be appreciated by those skilled in the art that the process of the present invention will be applicable in cases where any of X¹⁻⁴ is a non-reacting substituent, i.e. being stable under hydroformylation conditions, for example as taught by Cuny and Buchwald, J. Am. Chem. Soc., 1993, 115, 2066.

The catalyst for the process is selected such that the ratio of the product (a) to its branched regioisomer (**III**) is at least 80:20, more preferably is at least 90:10 and ideally is at least 98:2, or higher. Suitable catalysts for this purpose comprise a group VIII transition metal complexed to a phosphorus-containing ligand. Preferably, the transition metal is selected from the group consisting of rhodium (Rh), cobalt (Co), iridium (Ir), ruthenium (Ru), iron (Fe), nickel (Ni), palladium (Pd), platinum (Pt), and osmium (Os). More preferably the transition metal is either Rh, Co, Ir, or Ru and most preferably it is Rh. Preferably, the ligand is selected from the group comprising triorganophosphines, triorganophosphites, diorganophosphites, and bisphosphites. More preferably, the ligand is a bisphosphite, and typically contains the partial formula (**IV**). Representative ligands of this type, having utility in the process of the invention, are selected from the group including BIPHEPHOS (**V**), (**VI**) and the unsymmetrical bisphosphite (**VII**) in which R is H, CH₃, OCH₃, or OC₂H₅.

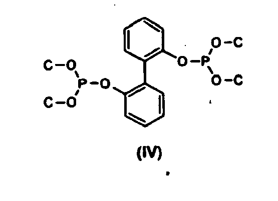

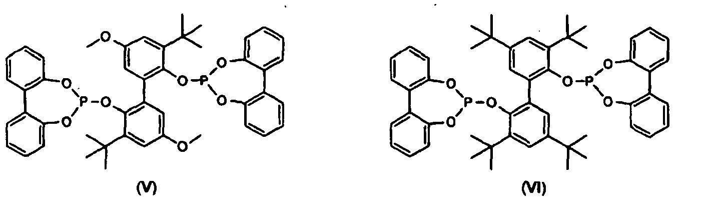

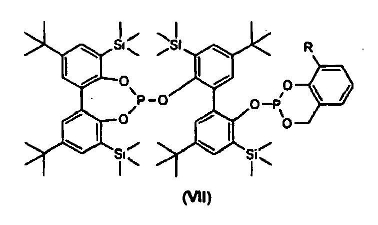

For operation of the process of the invention, a pre-formed, storage-stable complex of the transition metal and phosphorus-containing ligand may be employed, although more commonly, the catalyst complex is prepared in solution prior to use, and said solution is combined in the reaction vessel with a solution of the α-olefin substrate (**II**) and the syngas reagent. Preparation of the solution of catalyst complex entails reaction of the ligand with a precursor complex containing the transition metal, optionally using a molar excess of ligand such that uncomplexed ligand is present once all of the precursor complex is consumed. Additional ligand may also be added during the course of the hydroformylation reaction. Where the transition metal is Rh, the precursor complex is preferably Rh(acac)(CO)₂. Preferably the molar ratio of ligand:transition metal is in the range of about 1:1 to 100:1, and more preferably this ratio is in the range of about 1.3:1 to 3:1.

The reaction conditions for effecting hydroformylation of the α-olefin substrate (**II**) can be chosen from any of those conditions conventionally used and known for such processes. Generally, the hydroformylation process temperature is greater than about 25°C. preferably greater than about 35 °C, and more preferably greater then about 45°C. Generally, the hydroformylation process temperature is less than about 110°C, preferably less than about 100°C and more preferably less than about 90°C. The hydroformylation process may be conducted as a batch or continuous process. Preferably, the total pressure of hydrogen and carbon monoxide is less than about 2000 psia (13,790 kPa), and more preferably less than about 1500 psia (10,342 kPa). More specifically, the carbon monoxide partial pressure of the hydroformylation process of this invention is typically greater than about 10 psia (69 kPa), preferably greater than about 20 psia (138 kPa). The carbon monoxide partial pressure of the hydroformylation process of this invention is typically less than about 1000 psia (6,895 kPa), preferably less than about 750 psia (5171 kPa). The hydrogen partial pressure is typically greater than about 5 psia (35 kPa), preferably greater than about 10 psia (69 kPa). The hydrogen partial pressure is typically less than about 1000 psia (6,895 kPa), preferably less than about 750 psia (5171 kPa). In general, the H₂/CO molar ratio of gaseous hydrogen to carbon monoxide may be greater than about 1/10, and preferably, equal to or greater than about 1/1. The H₂/CO molar ratio may be less than about 100/1, and preferably, equal to or less than about 10/1.

The hydroformylation process of this invention is also preferably conducted in the presence of an organic solvent that solubilizes the Group VIII transition metal complex catalyst. Any suitable solvent or mixture of solvents that does not interfere unduly with the hydroformylation and product-catalyst separation process can be used, including those types of solvents commonly used in prior art hydroformylation processes. Isolation of aldehyde (**I**) can be accomplished by a non-aqueous phase separation procedure in which aldehyde (**I**) is extracted into a polar organic solvent and residual metal-containing complexes are extracted into a non-polar organic solvent. This procedure is described more fully in US5952530, the contents of which are incorporated herein by reference. Alternatively, the aldehyde (**I**) may by subjected to one or more downstream chemical processes, without prior isolation from the hydroformylation reaction mixture.

Another aspect of the present invention provides a novel composition, α-olefin of formula (**II**), wherein wherein (N)_{PrG} is a cyclic imide, R is an alkyl or aralkyl group and each of X¹⁻⁴ is independently H or a substituent that is unreactive under hydroformylation conditions. Preferably, R in compound (**II**) is selected from the group consisting of methyl, ethyl, n-propyl, *n*-butyl, benzyl and benzhydryl. More preferably, R is methyl. Preferably, (N)_{PrG} is *N*-phthalimide. Preferably, each of X¹⁻⁴ is H.

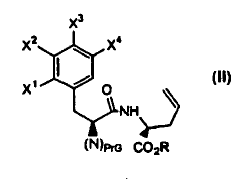

In a preferred embodiment, the process of the present invention enables an efficient synthetic route to MDL 28,726 (Scheme 3). Once the aldehyde (**Ia**) has been made, this route comprises treatment with one or more acid reagents to effect sequentially (i) conversion of aldehyde (**I**) to 5,6-didehydropipecolate (**IXa**) and (ii) cyclization of (**IXa**) to form MDL 28,726 or its carboxylic ester precursor. Preparation of the hydroformylation substrate, α-olefin (**IIa**) wherein R is methyl, is achieved by coupling of reagents (**X**) and (**XI**), derived from (*S*)-phenylalanine and (*S*)-allylglycine respectively. In the context of disclosures in the prior art, notable features of the overall synthetic route include the following:
(a) Swern oxidation is avoided.
(b) Complete control over all stereocentres is maintained throughout the synthesis.
(c) Surprisingly for the substrate (**IIa**) containing a methyl ester, a very high ratio linear:branched aldehyde products is observed, when a Rh-BIPHENPHOS complex is used as the catalyst. The presence of a *t*-butyl ester in the substrate is not required. The resultant methyl ester-containing product (**Ia**) has ideal solubility characteristics to enable clean separation from catalyst residues according to the non-aqueous phase separation procedure described above.

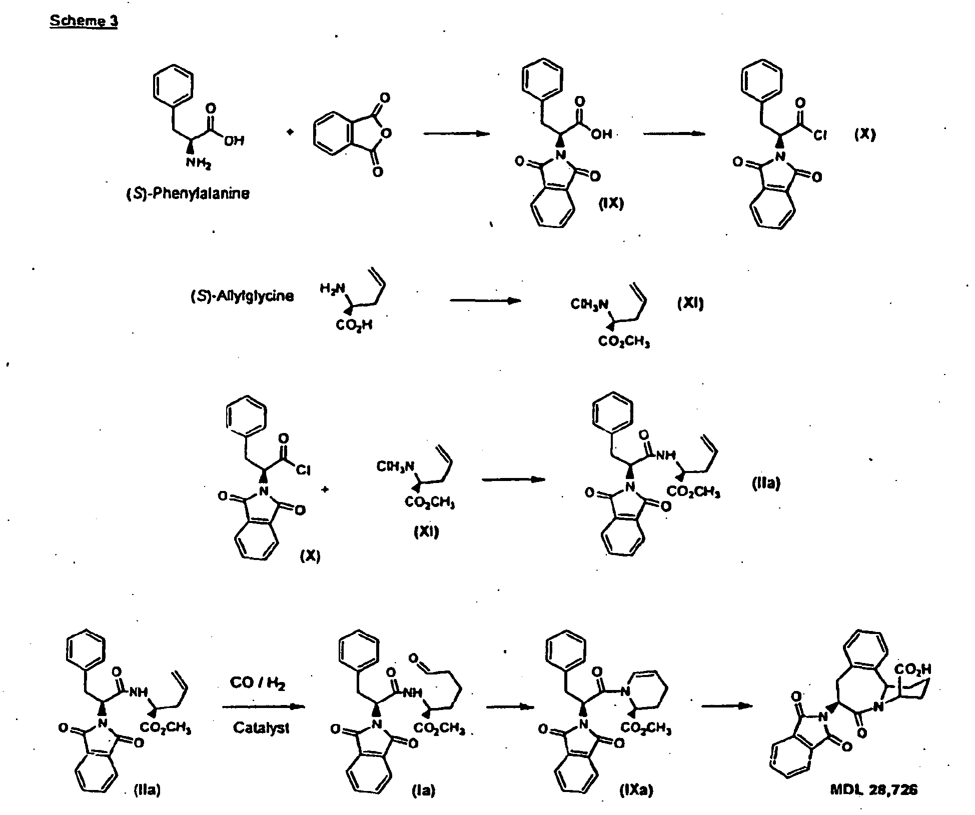

The invention is further illustrated by the following examples.

### Example 1 (Comparative)

### Preparation of N-phthaloyl (S)-phenylalanine acid chloride (X)

A 1-L round-bottom flask was charged with 50.19 g of (*S*)-phenylalanine, 47.3 g of phthalic anhydride, 0.5 mL of triethylamine, and 500 mL of toluene. The mixture was heated to reflux with stirring, and the water removed using a Dean Stark trap. After the water removal was complete, the mixture was cooled to ambient temperature, chilled in an ice bath, and the solid isolated by filtration to give a 103.4 g wet-cake of (**IX**) (80.3% solids, 83.0 g dry weight basis, 92.6% yield). A 60.47-g portion of the wet-cake was charged to a 1-L flask with 250 mL of toluene and 1 mL of *N,N*-dimethylformamide. To the slurry was added dropwise 19 mL of oxalyl chloride. After stirring oversight at ambient temperature, the solvent was evaporated to give a 139.7 g residue. The residue was dissolved in 200 g of ethyl acetate to give a 15 wt% solution of the acid chloride (**X**) in ethyl acetate.

### Example 2 (Comparative)

### Preparation of (S)-Allylglycine Methyl Ester Hydrochloride (XI)

A 250-mL round-bottom flask was charged 66.5 g of methanol, 4.45 g of anhydrous hydrogen chloride, and 3.16 g of (*S*)-allylglycine. The mixture was heated to reflux for one hour, then cooled for the addition of 10 mL of trimethylorthoformate. The solution was heated to reflux for six hours, then cooled to ambient temperature and diluted with 50 mL of toluene. The mixture was evaporated to a residue. An additional 50 mL of toluene was added, and the solvent evaporated to a residue of 5.58 g containing (*S*)-allylglycine methyl ester hydrochloride (**XI**).

### Example 3 (Comparative)

### Preparation of (S)-N-[2-(1,3-Dihydro-1.3-dioxo-2H-isoindol-2-yl)-1-oxo-3-phenylpropyl)-allyl-(S)-glycine Methyl Ester (IIa)

To the 5.58-g residue of (S)-allylglycine methyl ester hydrochloride (**XI**) prepared above was added 19 g of ethyl acetate and 6 g of acetonitrile. The mixture was chilled in an ice bath for the dropwise addition of 9.2 g of N-methylmorpholine. To the resulting mixture was added dropwise 63 g of the *N*-phthaloyl-(*S*)-phenylalanine acid chloride (**X**) in ethyl acetate solution prepared above. Hollowing reaction completion, the mixture was diluted with 20 mL of water. The pH was adjusted to 1 with 6.3 g of 37% hydrochloric acid, and the aqueous phase was removed. Water (25 mL) was added, and the pH adjusted to 8.5 by the addition of sodium bicarbonate. The aqueous phase was removed. The solvent was removed from the organic phase to give a 15.2 g residue. The residue was treated with 51 mL of 2-propanol, the mixture heated to reflux to give a solution, then cooled to give a slurry. The mixture was chilled in an ice bath and the solid collected by filtration, rinsed with 5 mL of 2-propanol, and dried at 40°C under vacuum to give 7.54 g (67% yield) of (*S*)-N-[2-(1,3-dihydro-1,3-dioxo-2H-isoindol-2-yl)-1-oxo-3-phenylpropyl)-allyl-(*S*)-glycine methyl ester (**IIa**). HPLC analysis indicated the purity was 99%.

### Example 4

### Preparation of (S)-2-[(S)-2-(1,3-dihydro-1.3-dioxo-2H-isoindol-2-yl)-1-oxo-3-phenylpropyl)-6-oxo-hexanoic acid methyl ester (Ia) using a Rh-(VII) complex as catalyst

In a glove box, a THF (8.00g) solution of Rh(acac)(CO)₂ (0.0188g, 0.0729mmol) and *N,N-*diisopropylethylamine (0.252g, 1.95mmol)) was prepared and added to the ligand (**VII**) wherein R is methoxy (0.100g, 0.0913mmol; W02004035595) in 7.00g THF. Compound (**IIa**) (7.00g, 17.2mmol) was dissolved in 15g THF. The catalyst solution was charged into the reactor and the compound (II) solution into a 35mL substrate feed cylinder. The reactor and the cylinder were purged with 1:1 syn gas (90psi) three times. The reactor was then pressurized with 1:1 syn gas to 40 psig and heated to 54°C. After stirring the catalyst solution at 54 °C for 15 minutes, the compound (II) solution was added into the reactor with 90 psi 1:1 syn gas. The reactor was then fed with 1:1 syn gas from a 310cc cylinder. Reaction time was recorded for every 6 psi gas uptake from the 310cc syn gas cylinder. After 1 hour and 43 minutes, gas uptake stopped. The reaction solution was collected into a 250 mL Schlenk flask containing 10mL of pentadecane under nitrogen. After evaporating the THF, acetonitrile (60mL) and pentane (60 mL) were added. After stirring for 5 minutes, the acetonitrile phase was separated and extracted with pentane (3X20mL). Acetonitrile was evaporated to obtain 5.3g (70%) off-white solid of (**Ia**). The product was characterized by ¹H and ¹³C NMR, and HPLC analysis. The ratio of linear to branched aldehyde was approximately 12:1.

### Example 5

### Preparation of (S)-2-[(S)-(1-3-dihydro-1,3-dioxo-2H-isoindol-2-yl)-1-oxo-3-phenylpropyl)-6-oxo-hexanoic acid methyl ester (Ia using a Rh-(V) complex as catalyst

In a glove box, a THF (8.00g) solution of Rh(acac)(CO)₂ (0.0149g, 0.0578mmol) was prepared and added to the solution of BIPHENPHOS (V; 0.0907g, 0.1153mmol) in 7.00g THF. Compound (**IIa**) (7.00g, 17.2mmol) was dissolved in 15g THF. The catalyst solution was charged into the reactor and the compound (II) solution into a 35mL substrate feed cylinder. The reactor and the cylinder were purged with 1:1 syn gas (70psi) three times. The reactor was then pressurized with 1:1 syn gas to 70 psi and heated to 65 °C. After stirring the catalyst solution at 65 °C for 15 minutes, the pressure in the reactor was reduced to 65psi and the compound (II) solution was added into the reactor with 70 psig 1:1 syn gas. The reactor was vented until all the feed solution was added into the reactor. The reactor was then fed with 1:1 syn gas from a 310cc cylinder. Reaction time was recorded for every 6 to 10psi gas uptake from the 310cc syn gas cylinder. After 2 hours, gas uptake stopped. The reaction solution was collected into a 250 mL Schlenk flask containing 10mL of pentadecane under nitrogen. After evaporating about 90% of the THF, acetonitrile (60mL) and pentane (60 mL) were added. After stirring for 5 minutes, the acetonitrile phase was separated and extracted with pentane (3X20mL). Acetonitrile was evaporated to obtain 6.04g (80%) white solid of (**Ia**). The product was characterized by ¹H and ¹³C NMR, analysis. The ratio of linear to branched aldehyde was approximately 99:1.

### Example 6

### Preparation of MDL 28,726

A 25-mL round-bottom flask was charged with 1.52 g of ((*S*)-2-[(S)-2-(1,3- dihydro-1,3-dioxo-2H-isoindol-2-yl)-1-oxo-3-phenylpropyl)-6-oxo-hexanoic acid methyl ester (**Ia**) and 2.7 g of ethyl acetate. One drop of methane sulfonic acid was added, and the mixture stirred at ambient temperature. After about one hour, a slurry had formed. The solid was isolated by filtration and rinsed with 0.5 mL of ethyl acetate and dried at 40 °C under vacuum to give 0.74 g (51% yield) of (*S*)-1-[(*S*)-2-(1,3-dihydro-1,3-dioxo-2H-isoindol-2-yl)-3-phenylpropionyl)]1,2,3,4-tetrahydro-pyridine-2-carboxylic acid methyl ester (**IXa**). HPLC analysis indicated the material was 98% pure. Methyl ester (**IXa**) can be converted to MDL 28,726 by further treatment with acid, for example according to the procedure described by Horgan et al. in Org. Proc. Res. Dev., 1999, 3, 241.

## Claims

1. A process for the preparation of an aldehyde of formula (**I**), wherein (N)_{PrG} is a protected amino group, R is an alkyl or aralkyl group and each of X¹⁻⁴ is independently H or a non-reacting substituent, which comprises hydroformylation of an α-olefin of formula (**II**), by reaction with syngas (CO/H₂) in the presence of, as catalyst, a group VIII transition metal complex of a phosphorus-containing ligand.

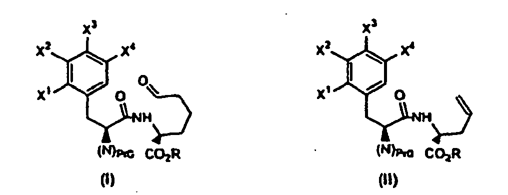

2. A process according to claim 1, wherein each of X¹⁻⁴ is H.

3. A process according to claim 1, wherein R is selected from the group consisting of methyl, ethyl, n-propyl, n-butyl, benzyl and benzhydryl.

4. A process according the claim 3, wherein R is methyl.

5. A process according to claim 1, wherein (N)_{PrG} is stable to acid treatment.

6. A process according to claim 5, wherein (N)_{PrG} is a cyclic imide.

7. A process according to claim 6, wherein (N)_{PrG} is *N*-phthalimide.

8. A process according to claim 1, wherein the ratio of the product (**I**) to its branched regioisomer (**III**) is at least 80:20.

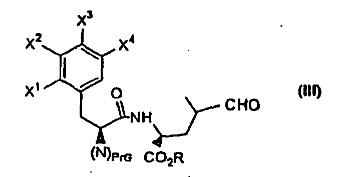

9. A process according to claim 8, wherein the ratio of the product (**I**) to its branched regioisomer (**III**) is at least 90: 10.

10. A process according to claim 9, wherein the ratio of the product (**I**) to its branched regioisomer (**III**) is at least 98:2.

11. A process according to claim 1, wherein the transition metal is selected from the group consisting of rhodium (Rh), cobalt (Co), iridium (Ir), ruthenium (Ru), iron (Fe), nickel (Ni), palladium (Pd), platinum (Pt), and osmium (Os).

12. A process according to claim 11, wherein the transition metal is selected from the group consisting of rhodium (Rh), cobalt (Co), iridium (Ir), ruthenium (Ru).

13. A process according to claim 12 wherein the transition metal is Rh.

14. A process according to claim 1, wherein the ligand is selected from the group comprising triorganophosphines, triorganophosphites, diorganophosphites, and bisphosphites.

15. A process according to claim 14, wherein the ligand is a bisphosphite.

16. A process according to claim 15, wherein the bisphosphite contains the partial formula (**IV**).

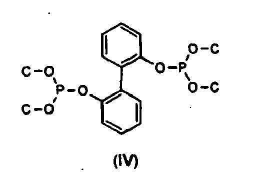

17. A process according to claim 16, wherein the bisphosphite is selected from the group consisting of compounds (**V**), (**VI**) and (**VII**) wherein R is H, CH₃, OCH₃, or OC₂H₅.

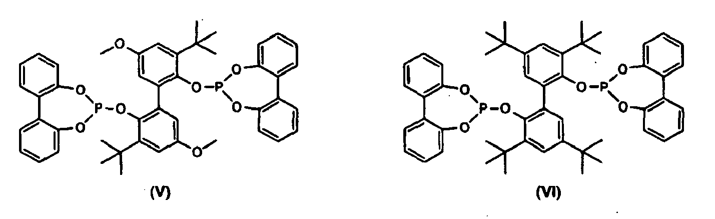

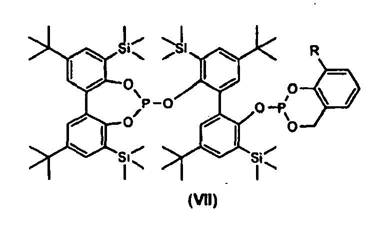

18. A process according to claim 17, wherein the bisphosphite is compound (**V**).

19. A process according to claim 1, wherein the catalyst is generated in the reaction vessel by reaction of the ligand with a precursor complex containing the transition metal, optionally using an molar excess of ligand such that uncomplexed ligand is present once all of the precursor complex is consumed.

20. A process according to claim 1, wherein the transition metal is Rh and the precursor complex is Rh(acac)(CO)₂.

21. A process according to claim 20, wherein the molar ratio of ligand:transition metal is in the range of about 1:1 to 100:1.

22. A process according to claim 21, wherein the molar ratio of ligand: transition metal is in the range of about 1.3:1 to 3:1.

23. A process according to claim 1, wherein the reaction temperature is in the range of about 25°C to 110°C.

24. A process according to claim 23, wherein the reaction temperature is in the range of about 45°C to 90°C.

25. A process according to any preceding claim, which further comprises conversion to a tricyclic acid of formula (**VIII**).

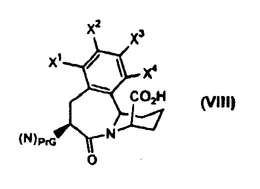

26. A process according to claim 25, wherein conversion to compound (**VIII**) comprises treatment with one or more acid reagents to effect sequentially (i) conversion of aldehyde (**I**) to 5,6-didehydropipecolate (**IX**) and (ii) cyclization of (**IX**) to form compound (**VIII**) or its carboxylic ester precursor.

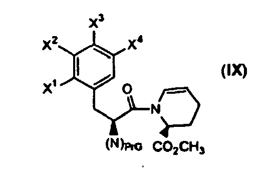

27. A process according to claim 26, wherein the aldehyde (**I**) is isolated from the hydroformylation reaction mixture prior to step (i).

28. A process according to claim 27, wherein the process to isolate aldehyde (**I**) comprises a non-aqueous phase separation procedure in which aldehyde (**I**) is extracted into a polar organic solvent and residual metal-containing complexes are extracted into a non-polar organic solvent.

29. A process according to claim 26, wherein the aldehyde (**I**) is not isolated from the hydroformylation reaction mixture prior to step (i).

30. A process according to claim 25, wherein the tricyclic acid is MDL 28,726

31. An α-olefin of according to formula (II) in claim 1, wherein (N)_{PrG} is a cyclic imide.

32. An α-olefin according to claim 31, wherein R is selected from the group consisting of methyl, ethyl, *n*-propyl, *n*-butyl, benzyl and benzhydryl.

33. An α-olefin according to claim 32, wherein R is methyl.

34. An α-olefin according to claim 33, wherein (N)_{PrG} is stable to acid treatment.

35. An α-olefin according to claim 34, wherein (N)_{PrG} is a *N*-phthalimide.

## Patentansprüche

1. Verfahren zur Herstellung eines Aldehyds der Formel (I), worin (N)_{PrG} eine geschützte Aminogruppe ist, R eine Alkyl- oder Aralkylgruppe ist und jeder von X¹⁻⁴ unabhängig voneinander H oder ein nicht-reagierender Substituent ist, das die Hydroformylierung eines α-Olefins der Formel (II) durch die Reaktion mit Syngas (CO/H₂) in Anwesenheit eines Gruppe VIII Übergangsmetallkomplexes eines phosphorhaltigen Liganden als Katalysator umfasst.

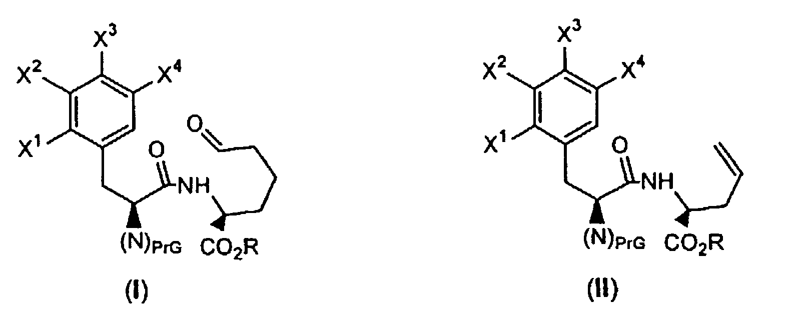

2. Verfahren nach Anspruch 1, worin jeder von X¹⁻⁴ H ist.

3. Verfahren nach Anspruch 1, worin R ausgewählt ist aus der Gruppe bestehend aus Methyl, Ethyl, *n*-Propyl, *n*-Butyl, Benzyl und Benzhydryl.

4. Verfahren nach Anspruch 3, worin R Methyl ist.

5. Verfahren nach Anspruch 1, worin (N)_{PrG} gegenüber Säurebehandlung stabil ist.

6. Verfahren nach Anspruch 5, worin (N)_{PrG} ein zyklisches Imid ist.

7. Verfahren nach Anspruch 6, worin (N)_{PrG} *N*-Phthalimid ist.

8. Verfahren nach Anspruch 1, worin das Verhältnis des Produkts (I) zu seinem verzweigten Regioisomer (III) mindestens 80:20 ist.

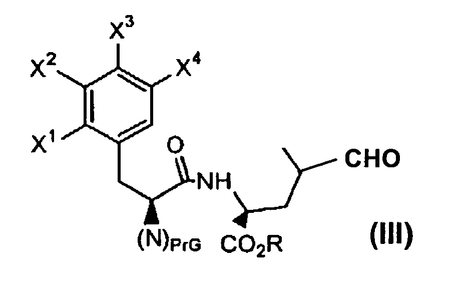

9. Verfahren nach Anspruch 8, worin das Verhältnis des Produkts (I) zu seinem verzweigten Regioisomer (III) mindestens 90:10 ist.

10. Verfahren nach Anspruch 9, worin das Verhältnis des Produkts (I) zu seinem verzweigten Regioisomer (III) mindestens 98:2 ist.

11. Verfahren nach Anspruch 1, worin das Übergangsmetall ausgewählt ist aus der Gruppe bestehend aus Rhodium (Rh), Cobalt (Co), Iridium (Ir), Ruthenium (Ru), Eisen (Fe), Nickel (Ni), Palladium (Pd), Platin (Pt) und Osmium (Os).

12. Verfahren nach Anspruch 11, worin das Übergangsmetall ausgewählt ist aus der Gruppe bestehend aus Rhodium (Rh), Cobalt (Co), Iridium (Ir), Ruthenium (Ru).

13. Verfahren nach Anspruch 12, worin das Übergangsmetall Rh ist.

14. Verfahren nach Anspruch 1, worin der Ligand ausgewählt ist aus der Gruppe umfassend Triorganophosphine, Triorganophosphite, Diorganophosphite und Bisphosphite.

15. Verfahren nach Anspruch 14, worin der Ligand ein Bisphosphit ist.

16. Verfahren nach Anspruch 15, worin das Bisphosphit die Teilformel (IV) enthält.

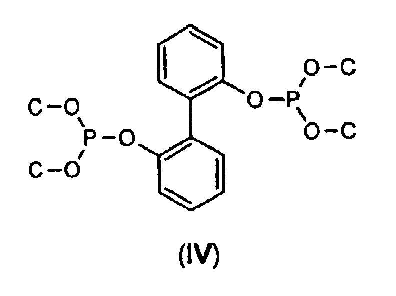

17. Verfahren nach Anspruch 16, worin das Bisphosphit ausgewählt ist aus der Gruppe bestehend aus den Verbindungen (V), (VI) und (VII), worin R H, CH₃, OCH₃ oder OC₂H₅ ist.

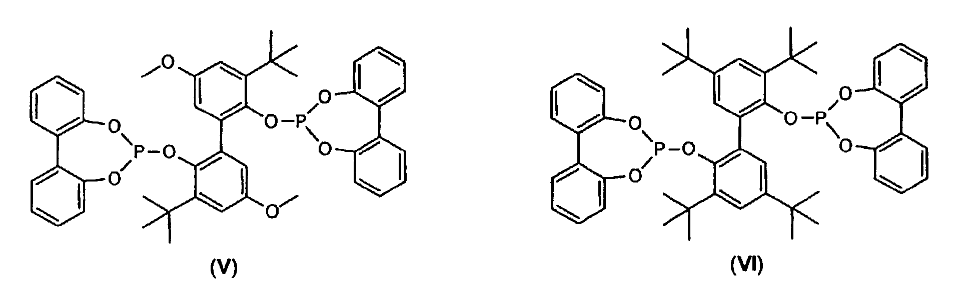

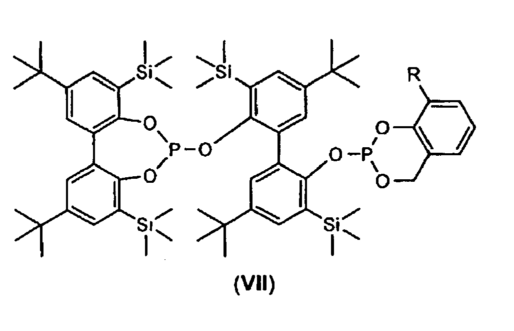

18. Verfahren nach Anspruch 17, worin das Bisphosphit die Verbindung (V) ist.

19. Verfahren nach Anspruch 1, worin der Katalysator im Reaktionsgefäß durch die Reaktion des Liganden mit einem Vorläuferkomplex, der das Übergangsmetall enthält erzeugt wird, gegebenenfalls unter Verwendung eines molaren Überschusses des Liganden so, dass Ligand nicht-komplexiert vorliegt, wenn der gesamte Vorläuferkomplex verbraucht ist.

20. Verfahren nach Anspruch 1, worin das Übergangsmetall Rh ist und der Vorläuferkomplex Rh(acac)(CO)₂ ist.

21. Verfahren nach Anspruch 20, worin das Molverhältnis von Ligand:Übergangsmetall im Bereich von etwa 1:1 bis 100:1 ist.

22. Verfahren nach Anspruch 21, worin das Molverhältnis von Ligand:Übergangsmetall im Bereich von etwa 1,3:1 bis 3:1 ist.

23. Verfahren nach Anspruch 1, worin die Reaktionstemperatur im Bereich von etwa 25°C bis 110°C ist.

24. Verfahren nach Anspruch 23, worin die Reaktionstemperatur im Bereich von etwa 45°C bis 90°C ist.

25. Verfahren nach einem der vorhergehenden Ansprüche, das weiter die Umwandlung in eine trizyklische Säure der Formel (VIII) umfasst.

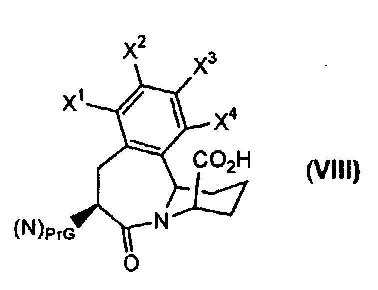

26. Verfahren nach Anspruch 25, worin die Umwandlung zur Verbindung (VIII) die Behandlung mit einem oder mehreren Säure-Reagenz(ien) umfasst, um aufeinanderfolgend (i) die Umwandlung von Aldehyd (I) zu 5,6-Didehydropipecolat (IX) und (ii) die Zyklisierung von (IX) zu bewirken, um die Verbindung (VIII) oder ihre Carbonsäureester-Vorläufer zu bilden.

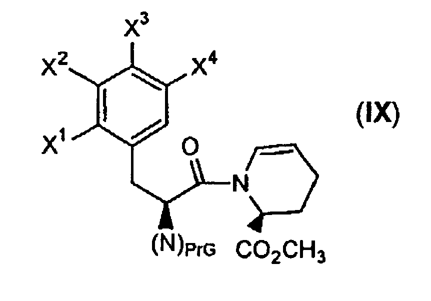

27. Verfahren nach Anspruch 26, worin das Aldehyd (I) aus der Hydroformylierungsreaktionsmischung vor Schritt (i) isoliert wird.

28. Verfahren nach Anspruch 27, worin das Verfahren zur Isolation von Aldehyd (I) ein nicht-wässeriges Phasentrennungsverfahren umfasst, in dem das Aldehyd (I) in ein polares organisches Lösungsmittel extrahiert wird und die restlichen Metall-enthaltenden Komplexe in ein nicht-polares organisches Lösungsmittel extrahiert werden.

29. Verfahren nach Anspruch 26, worin das Aldehyd (I) nicht aus der Hydroformylierungsreaktionsmischung vor Schritt (i) isoliert wird.

30. Verfahren nach Anspruch 25, worin die trizyklische Säure MDL 28,726 ist

31. α-Olefin nach Formel (II) in Anspruch 1, worin (N)_{PrG} ein zyklisches Imid ist.

32. α-Olefin nach Anspruch 31, worin R ausgewählt ist aus der Gruppe bestehend aus Methyl, Ethyl, *n*-Propyl, *n*-Butyl, Benzyl und Benzhydryl.

33. α-Olefin nach Anspruch 32, worin R Methyl ist.

34. α-Olefin nach Anspruch 33, worin (N)_{PrG} gegenüber Säurebehandlung stabil ist.

35. α-Olefin nach Anspruch 34, worin (N)_{PrG} ein *N*-Phthalimid ist.

## Revendications

1. Procédé de préparation d'un aldéhyde de formule (I), dans laquelle (N)_{PrG} représente un groupe amino protégé, R représente un groupe alkyle ou aryl-alkyle, et chacun des symboles X¹ à X⁴ représente indépendamment un atome d'hydrogène ou un substituant non-réagissant, lequel procédé comporte l'hydroformylation d'une α-oléfine de formule (II), effectuée par réaction de celle-ci avec du gaz de synthèse (CO/H₂) en présence, en tant que catalyseur, d'un complexe d'un métal de transition du groupe VIII et d'un ligand phosphoré.

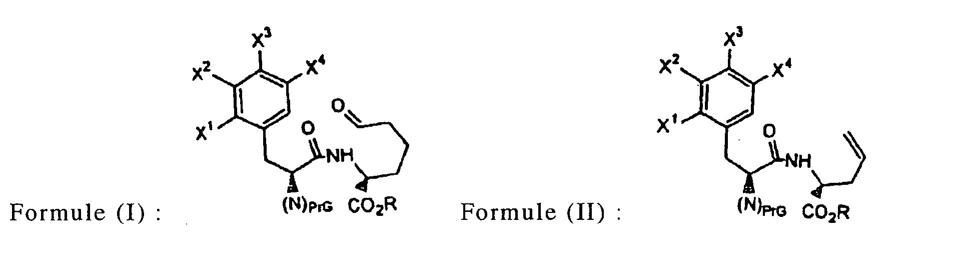

2. Procédé conforme à la revendication 1, dans lequel chacun des symboles X¹ à X⁴ représente un atome d'hydrogène.

3. Procédé conforme à la revendication 1, dans lequel R représente un groupe choisi dans l'ensemble constitué par les groupes méthyle, éthyle, n-propyle, n-butyle, benzyle et benzhydryle.

4. Procédé conforme à la revendication 3, dans lequel R représente un groupe méthyle.

5. Procédé conforme à la revendication 1, dans lequel le groupe représenté par (N)_{PrG} est stable vis-à-vis d'un traitement par un acide.

6. Procédé conforme à la revendication 5, dans lequel (N)_{PrG} représente un groupe de type imide cyclique.

7. Procédé conforme à la revendication 6, dans lequel (N)_{PrG} représente un groupe N-phtalimido.

8. Procédé conforme à la revendication 1, dans lequel le rapport du produit de formule (I) à son régio-isomère ramifié de formule (III) vaut au moins 80/20.

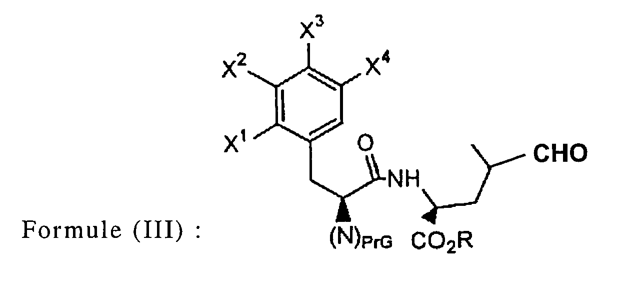

9. Procédé conforme à la revendication 8, dans lequel le rapport du produit de formule (I) à son régio-isomère ramifié de formule (III) vaut au moins 90/10.

10. Procédé conforme à la revendication 9, dans lequel le rapport du produit de formule (I) à son régio-isomère ramifié de formule (III) vaut au moins 98/2.

11. Procédé conforme à la revendication 1, dans lequel le métal de transition est choisi dans l'ensemble constitué par les rhodium (Rh), cobalt (Co), iridium (Ir), ruthénium (Ru), fer (Fe), nickel (Ni), palladium (Pd), platine (Pt) et osmium (Os).

12. Procédé conforme à la revendication 11, dans lequel le métal de transition est choisi dans l'ensemble constitué par les rhodium (Rh), cobalt (Co), iridium (Ir) et ruthénium (Ru).

13. Procédé conforme à la revendication 12, dans lequel le métal de transition est du rhodium (Rh).

14. Procédé conforme à la revendication 1, dans lequel le ligand est choisi dans l'ensemble comprenant les triorgano-phosphines, les tri-organo-phosphites, les diorgano-phosphites et les bisphosphites.

15. Procédé conforme à la revendication 14, dans lequel le ligand est un bisphosphite.

16. Procédé conforme à la revendication 15, dans lequel le bisphosphite comprend un fragment de formule (IV).

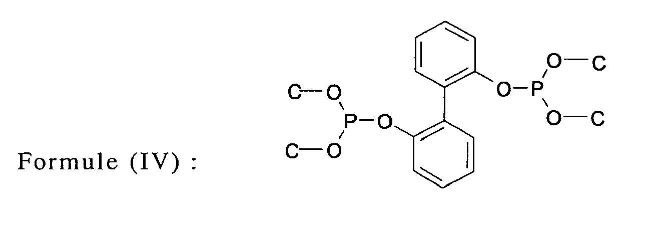

17. Procédé conforme à la revendication 16, dans lequel le bisphosphite est choisi dans l'ensemble constitué par les composés de formules (V), (VI) et (VII), dans laquelle formule (VII) R représente un atome d'hydrogène ou un groupe méthyle, méthoxy ou éthoxy.

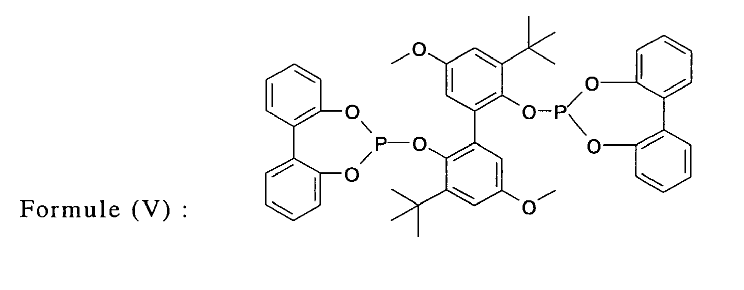

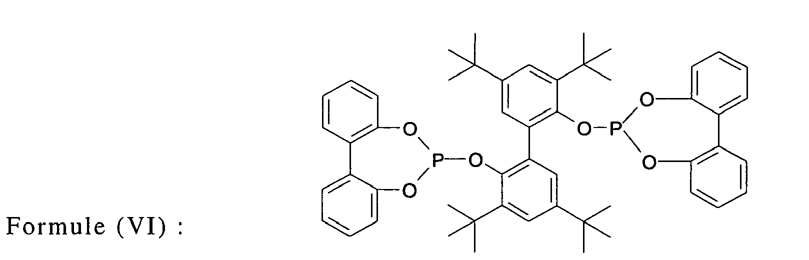

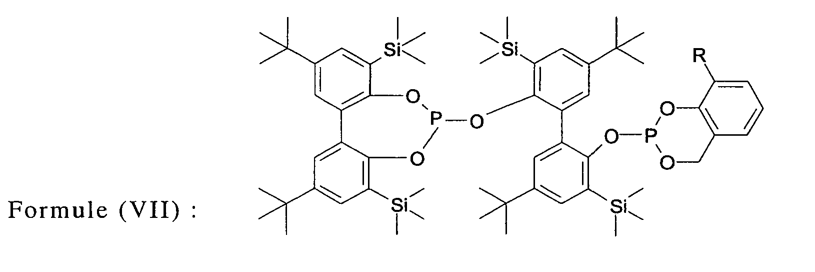

18. Procédé conforme à la revendication 17, dans lequel le bisphosphite est le composé de formule (V).

19. Procédé conforme à la revendication 1, pour lequel on génère le catalyseur dans le récipient réactionnel, en faisant réagir le ligand et un complexe précurseur contenant le métal de transition, et en employant, en option, le ligand en excès molaire, de telle sorte qu'il y a du ligand qui se trouve encore à l'état non-complexé après consommation de la totalité du complexe précurseur.

20. Procédé conforme à la revendication 1, dans lequel le métal de transition est du rhodium (Rh) et le complexe précurseur est le complexe de formule Rh(acac)(CO)₂.

21. Procédé conforme à la revendication 20, dans lequel le rapport molaire du ligand au métal de transition vaut d'environ 1/1 à 100/1.

22. Procédé conforme à la revendication 21, dans lequel le rapport molaire du ligand au métal de transition vaut d'environ 1,3/1 à 3/1.

23. Procédé conforme à la revendication 1, dans lequel la température de réaction vaut d'environ 25 à 110 °C.

24. Procédé conforme à la revendication 23, dans lequel la température de réaction vaut d'environ 45 à 90 °C.

25. Procédé conforme à l'une des revendications précédentes, qui comporte en outre une conversion en un acide tricyclique de formule (VIII).

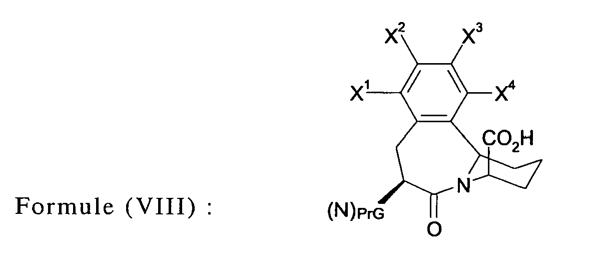

26. Procédé conforme à la revendication 25, dans lequel la conversion en un composé de formule (VIII) comprend des opérations de traitement effectuées avec un ou plusieurs réactif(s) acide(s) dans le but de réaliser successivement :
i) la conversion de l'aldéhyde de formule (I) en un 5,6-didéhydro-pipécolate de formule (IX),
ii) et la cyclisation de ce composé de formule (IX), ce qui donne un com-posé de formule (VIII) ou un précurseur d'un tel composé, de type ester d'acide carboxylique.

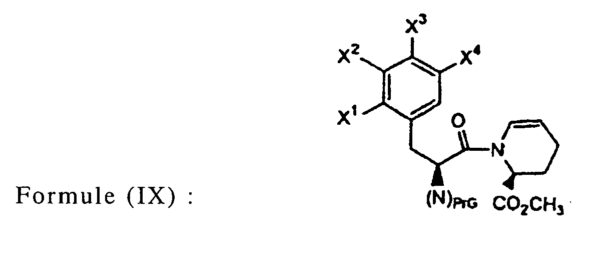

27. Procédé conforme à la revendication 26, dans lequel, avant d'effectuer l'étape (i), on isole l'aldéhyde de formule (I) en le séparant du mélange réactionnel d'hydroformylation.

28. Procédé conforme à la revendication 27, dans lequel l'isole-ment de l'aldéhyde de formule (I) comporte une opération de séparation en phases non-aqueuses, dans laquelle on extrait l'aldéhyde de formule (I) avec un solvant organique polaire et l'on extrait les résidus de complexes de métal avec un solvant organique non-polaire.

29. Procédé conforme à la revendication 26, dans lequel, avant d'effectuer l'étape (i), on ne sépare pas l'aldéhyde de formule (I) du mélange réactionnel d'hydroformylation.

30. Procédé conforme à la revendication 25, dans lequel l'acide tricyclique est le composé MDL 28726.

31. Alpha-oléfine de formule (II) indiquée dans la revendication 1, dans laquelle (N)_{PrG} représente un groupe de type imide cyclique.

32. Alpha-oléfine conforme à la revendication 31, dans laquelle R représente un groupe choisi dans l'ensemble constitué par les groupes méthyle, éthyle, n-propyle, n-butyle, benzyle et benzhydryle.

33. Alpha-oléfine conforme à la revendication 32, dans laquelle R représente un groupe méthyle.

34. Alpha-oléfine conforme à la revendication 33, dans laquelle le groupe représenté par (N)_{PrG} est stable vis-à-vis d'un traitement par un acide.

35. Alpha-oléfine conforme à la revendication 34, dans laquelle (N)_{PrG} représente un groupe N-phtalimido.
